# EUROPÄISCHE PATENTSCHRIFT

(11) **EP 1 501 775 B1**
(45) Veröffentlichungstag und Bekanntmachung des Hinweises auf die Patenterteilung: **29.12.2010**
(21) Anmeldenummer: 03718790.3
(22) Anmeldetag: 25.04.2003
(51) Int. Cl.: C07C 43/11, C11D 1/825

(54) **ALKOXYLATGEMISCHE UND DIESE ENTHALTENDE WASCHMITTEL**
ALKOXYLATE MIXTURES AND DETERGENTS CONTAINING THE SAME
MELANGES D'ALKOXYLATE ET LESSIVES LES CONTENANT

(30) Priorität: 26.04.2002 DE 10218752; 18.09.2002 DE 10243361
(43) Veröffentlichungstag der Anmeldung: 02.02.2005
(73) Patentinhaber: BASF SE, 67056 Ludwigshafen (DE)
(72) Erfinder: RULAND, Alfred, 69198 Schriesheim (DE); SCHOLTISSEK, Martin, 67157 Wachenheim (DE); TROPSCH, Jürgen, 67354 Römerberg (DE); BAUR, Richard, 67112 Mutterstadt (DE)
(86) Internationale Anmeldenummer: PCT/EP2003/004333
(87) Internationale Veröffentlichungsnummer: WO 2003/091190

(56) Entgegenhaltungen:
- EP-A- 0 616 026
- WO-A-01/77276
- WO-A-91/09925
- WO-A-92/14808
- WO-A-93/18188
- DE-A- 2 448 532
- DE-A- 4 237 178

## Beschreibung

Die Erfindung betrifft Alkoxylatgemische und diese enthaltende Waschmittel wie auch Verfahren zur Herstellung der Alkoxylatgemische und die Verwendung des Waschmittels zum Waschen oder Reinigen von Textilien.

Waschmittel im Sinne dieser Erfindung dienen in der Regel zum Waschen von mehr oder weniger flexiblen Materialien, vorzugsweise solchen, die natürliche, synthetische oder halbsynthetische Fasermaterialien enthalten oder daraus bestehen und die demzufolge zumindest teilweise einen textilen Charakter aufweisen.

Waschmittel dieser Art sind im Stand der Technik vielfach beschrieben worden. Einen sehr guten Überblick über die Wirkungsweise und die Zusammensetzung von Waschmitteln findet man beispielsweise in Ullmann's Encyclopedia of Industrial Chemistry, 5. Auflage, Bd. A8, (1986), Seiten 315 ff, Stichwort "Detergents" sowie in Tai, Formulating Detergents and Personal Care Products, AOCS Press, 2000. Die Waschmittel enthalten ein Tensid oder mehrere Tenside aus gleichen oder unterschiedlichen Tensidgruppen und in der Regel weitere Hilfs- und Zusatzstoffe, die entweder zur Konfektionierung erforderlich sind und/oder die einer Anpassung der Waschmittel an den geplanten speziellen Verwendungszweck oder die Art der Anwendung (Waschen von Hand oder in Maschinen) dienen. Bestandteile, die neben den verschiedenen Tensiden in wechselnden Kombinationen und Anteilen in vielen Waschmitteln eingesetzt werden können, sind zum Beispiel Builder (Sequestrierungsmittel) und Co-Builder, pH-Regulatoren, wie anorganische oder organische Säuren, anorganische oder organische Basen und Puffersysteme, Ionenaustauscher, Dispergiermittel, Schmutztragemittel, Verdickungsmittel, Enzyme, Bleichsysteme, hydrotrope Verbindungen als Lösungsvermittler bzw. Solubilisatoren, wie Harnstoff oder Alkohole, Schaumregulatoren zur Stabilisierung oder Dämpfung des Schaums, Haut- und Korrosionsschutzmittel, desinfizierende Verbindungen oder Systeme, beispielsweise solche, die Iod enthalten oder die Chlor oder unterchlorige Säure freisetzen, wie Dichlorisocyanurat, Parfüm, Farbstoffe, optische (fluoreszierende) Aufheller, Vergrauungsinhibitoren, Stell- und Konfektionierungsmittel und desinfizierende Verbindungen. Wesentlichen Anteil an der Reinigungswirkung der im Stand der Technik beschriebenen Waschmittel haben die darin enthaltenen Tenside. Verwendung finden ionische Tenside und zwar sowohl anionische Tenside wie beispielsweise Alkoholsulfate, Alkoholethersulfate, Alkylbenzolsulfonate, α-Olefinsulfonate, Sulfosuccinate als auch kationische Tenside, wie beispielsweise C₈ bis C₁₆-Dialkyldimethylammoniumsalze, Dialkoxydimethylammoniumsalze oder Imidazoliniumsalze mit langkettigem Alkylrest.

Auch der Einsatz von amphoteren Tensiden, beispielsweise von Derivaten von sekundären oder tertiären Aminen wie C₆-C₁₈-Alkylbetainen oder C₆-C₁₅-Alkylsulfobetainen oder Aminoxiden wie Alkyldimethylaminoxiden ist bereits beschrieben worden.

Auch nichtionische Tenside, insbesondere auch Alkoxylate und Polyglycoside von Alkanolen mit insbesondere 8 bis 20 C-Atomen sowie Alkoxylaten von Alkylaminen und Alkylamiden werden in Waschmitteln eingesetzt. Es ist insbesondere auch bekannt, Alkoxylate von Oxoalkoholen mit 10 bis 13 C-Atomen als Tenside in Waschmitteln einzusetzen. In der DE-A-100 29 692 sind derartige Alkoxylate beschrieben.

DE 24 48 532 A offenbart Gemische von Tensiden bestehend aus primären und sekundären C₈₋₁₅-Alkoholen mit verschiedenen Anteilen an Ethylenoxid. Eine Mischung enthaltend 2-Propyl-heptanol als Basisalkohol wird in diesem Dokument nicht genannt.

Im Interesse eines möglichst sparsamen Stoffeinsatzes, hoher Wirtschaftlichkeit und geringer Umweltbelastung streben die Waschmittelhersteller nach einer stetigen Verbesserung der Wirksamkeit ihrer Produkte und insbesondere der darin enthaltenen Tenside.

Aufgabe der vorliegenden Erfindung ist die Bereitstellung von Alkoholalkoxylat-Tensidsystemen, die in Wasch- und Reinigungsmitteln zu einer verbesserten Schmutzentfernung führten und das Leistungsspektrum der Wasch- und Reinigungsmittel verbessern.

Die Aufgabe wird erfindungsgemäß gelöst durch ein Alkoxylatgemisch, enthaltend
10 bis 90 Gew.% mindestens eines Alkoxylats der allgemeinen Formel (I)

C₅H₁₁CH(C₃H₇)CH₂O(A)ₓH (I)

mit der Bedeutung
- A: Ethylenoxy
- x: Zahl von 3 bis 12, und
10 bis 90 Gew.-% mindestens eines Alkoxylats der allgemeinen Formel (II)

CₘH₂ₘ₊₁O(A)ᵥ(B)_{w}H (II)

mit der Bedeutung
A Ethylenoxy
B C₃-C₁₀-Akylenoxy, vorzugsweise Propylenoxy, Butylenoxy, Pentylenoxy oder Gemische davon,
wobei Gruppen A und B statistisch verteilt, alternierend oder in Form zweier oder mehrerer Blöcke in beliebiger Reihenfolge vorliegen können,
- m: ganze Zahl im Bereich von 12 bis 24,
- v: Zahl im Bereich von 3 bis 15,
- w: 0, wobei im Alkoxylat der allgemeinen Formel (I)

| | |
|---|---|
| 85 bis 96 Gew.% | Alkoxylate A1, in denen C₅H₁₁ die Bedeutung n-C₅H₁₁ hat, und |
| 4 bis 15 Gew.-% | Alkoxylate A2, in denen C₅H₁₁ die Bedeutung C₂H₅CH(CH₃)CH₂ und/oder CH₃CH(CH₃)CH₂CH₂ hat, |

im Gemisch vorliegen.

Es wurde erfindungsgemäß gefunden, dass die Alkoxylatgemische, die sich von kürzerkettigen und längerkettigen Alkanolen ableiten, ein gegenüber bekannten Systemen deutlich verbessertes Waschverhalten zeigen. Die Verbesserung ist insbesondere im Vergleich zum Einsatz von ausschließlich kurzkettigen Alkanolethoxylaten deutlich. Die Verwendung derartiger kurzkettiger Alkanolalkoxylate in Detergenzzusammensetzungen ist an sich bekannt.

WO 94/11331 betrifft die Verwendung von Alkoxylaten von 2-Propylheptanol in Detergenzzusammensetzungen zur Entfettung harter Oberflächen. Die Alkoxylate weisen 2 bis 16 Alkylenoxid-Gruppen auf. Vorzugsweise liegt der überwiegende Teil der Alkylenoxid-Gruppen in Form von Ethylenoxid vor. Gemäß der Beispiele werden ausschließlich ethoxylierte Alkohole eingesetzt. Es ist ferner beschrieben, dass die Alkohole zunächst mit Ethylenoxid und sodann mit Propylenoxid umgesetzt werden können. Für derartige Alkoxylate sind jedoch keine Beispiele oder Eigenschaften angegeben. Es wird ausgeführt, dass die beschriebenen Alkoxylate eine gute Detergenzund Benetzungswirkung zeigen, verbunden mit einem geringen Schäumen. Zudem wird angegeben, dass die Alkoxylate einen erwünschten Verdickungseffekt in Formulierungen haben.

WO 94/11330 betrifft Alkoxylate von 2-Propylheptanol und deren Verwendung. In den Alkoxylaten liegt 2-Propylheptanol, zunächst mit 1 bis 6 mol Propylenoxid und sodann mit 1 bis 10 mol Ethylenoxid umgesetzt, vor. Gemäß den Beispielen wird ein zunächst mit 4 mol Propylenoxid und sodann mit 6 mol Ethylenoxid umgesetztes 2-Propylheptanol eingesetzt. Es wird angegeben, dass die Alkylenoxidaddukte ein verbessertes Verhältnis von Schaumverhalten zu Detergenzwirkung zeigen. Ferner ist angegeben, dass die Alkoxylate ein gutes Benetzungsverhalten zeigen. Sie werden in Detergenzzusammensetzungen zur Reinigung von Textilmaterialien eingesetzt.

In der US 2,508,036 sind Ethoxylate von 2-n-Propylheptanol mit 5 bis 15 mol Ethylenoxid beschrieben. Es ist angegeben, dass die Alkoxylate ein verbessertes Benetzungsverhalten in wässrigen Lösungen zeigen und daher in Waschmitteln in Kombination mit Buildern eingesetzt werden können.

Im folgenden werden die kurzkettige und die langkettige Alkanolalkoxylatkomponente der erfindungsgemäßen Alkoxylatgemische näher erläutert.

Die erfindungsgemäßen Gemische enthalten 10 bis 90 Gew.-%, insbesondere 20 bis 70 Gew.-% mindestens eines Alkoxylats der allgemeinen Formel (I). Entsprechend enthalten sie 10 bis 90 Gew.-%, insbesondere 30 bis 80 Gew.-% mindestens eines Alkoxylats der allgemeinen Formel (II).

Im Alkoxylat der allgemeinen Formel (I) hat A die Bedeutung Ethylenoxy.

Dabei hat C₃H₇ im Alkoxylat der allgemeinen Formel (I) vorzugsweise die Bedeutung n-C₃H₇.

Die Herstellung von 2-Propylheptanol(en) kann ausgehend von Valeraldehyd durch Aldolkondensation und nachfolgende Hydrierung erfolgen. Die Herstellung von Valeraldehyd und den entsprechenden Isomeren erfolgt durch Hydroformylierung von Buten, wie beispielsweise in US 4,287,370; Beilstein E IV 1, 3268, Ullmanns Encyclopedia of Industrial Chemistry, 5. Auflage, Band A1, Seiten 323 und 328f beschrieben. Die nachfolgende Aldolkondensation ist beispielsweise beschrieben in US 5,434,313. Die Hydrierung des Aldolkondensationsprodukt folgt allgemeinen Hydrierbedingungen.

Des weiteren kann 2-Propylheptanol durch Kondensation von 1-Pentanol (als Mischung der entsprechenden Methylbutanole-1) in Gegenwart von KOH bei erhöhten Temperaturen hergestellt werden, siehe z. B. Marcel Guerbet, C. R. Acad Sei Paris 128, 511, 1002 (1899). Des weiteren ist auf Römpp, Chemie Lexikon, 9. Auflage, Georg Thieme Verlag Stuttgart, und die dort genannten Zitate sowie Tetrahedron 1967, Vol. 23, Seiten 1723 - 1733, hinzuweisen.

Geeignete einfach verzweigte Alkylreste sind auch 2-Octyl-, 3-Octyl- usw., 2-Nonyl-, 3-Nonyl- usw., 2-Decyl-, 3-Decyl- usw., 2-Undecyl-, 3-Undecylreste usw. Entsprechende Alkohole können durch Addition von Wasser an Olefine, z. B. alpha-Olefine, hergestellt werden.

Geeignete mehrfach verzweigte Alkylreste enthalten einen oder zwei, vorzugsweise einen Methyl- oder Ethylsubstituenten. Ein Beispiel ist der (6-Ethyl) - 2 - nonylrest. Der entsprechende Alkohol ist durch Umsetzung von 2-Ethylhexanal mit Aceton und ausschießende Hydrierung zugänglich.

In der allgemeinen Formel (I) ist x eine Zahl von 3 bis 12. Die Werte von x stellen Mittelwerte dar, da bei der Alkoxylierung von Alkanolen in der Regel eine Verteilung des Alkoxylierungsgrades erhalten wird. Daher kann x, wie auch die nachstehend diskutierten v und w von ganzzahligen Werten abweichen. Die Verteilung des Alkoxylierungsgrades kann in gewissem Umfang durch Einsatz unterschiedlicher Alkoxylierungskatalysatoren eingestellt werden. Der Mittelwert der Homologenverteilung wird durch die angegebene Zahl x dargestellt.

Im längerkettigen Alkoxylat der allgemeinen Formal (II) haben A und B vorzugsweise die vorstehende Bedeutung. m ist eine ganze Zahl im Bereich von 12 bis 24, vorzugsweise von 12 bis 18, besonders bevorzugt von 12 bis 15. Der Alkyrest CₘH₂ₘ₊₁ kann dabei linear oder einfach oder mehrfach verzweigt sein. Es können auch Gemische von linearen und verzweigten Alkylresten vorliegen. Sie können aus beliebigen geeigneten Quellen stammen. Die linearen Alkohole sind nativen oder synthetischen Ursprungs. Unter den verzweigten Alkoholen sind beispielsweise zu nennen:
- Oxoalkohole auf Basis von linearen oder verzweigten Olefinen,
- sekundäre Alkohole, beispielsweise gewonnen durch Paraffinoxidation,
- Alkohole, gewonnen durch den SHOP-Prozess,
- Alkohole, erhältlich über die GTL-Technologie,
- Alkohole, erhältlich über die Fischer-Tropsch-Technologie,
- Alkohole, erhältlich über Gerüstisomerisierung der zur Oxierung eingesetzten Olefine entsprechend WO 98/23566.

Geeignete Alkohole, die verzweigt sind, weisen die Hydroxylgruppe z. B. in 2-, 3-, 4-Position usw. auf. Der Alkylrest kann linear oder nochmals verzweigt sein und z. B. Methyl- oder Ethylsubstituenten tragen.

Beispiele geeigneter Alkohole sind 2-Dodecanol, 2-Tetradecanol, 2-Hexadecanol, jeweils zugänglich durch Addition von Wasser an α-Olefine, (7-Ethyl)- 3-decanol bzw. (3-Methyl - 6 - ethyl)- 2-nonanol, erhältlich durch Umsetzung von 2-Ethyl-hexanal mit Methylethylketon und anschließende Hydrierung, 2-Hexadecanol bzw. 2-Octadecanol, erhältlich durch Umsetzung von C₁₃/C₁₅- Aldehyd mit Aceton, 3- Nonadecanol bzw. (3-Methyl) - 2- octadecanol, (3-Methyl) - 2 - hexadecanol, 3- Heptadecanol, erhältlich durch Umsetzung von C₁₃/C₁₅- Aldhyd mit Methylethylketon. Die Umsetzungsprodukte auf Basis von C₁₃/C₁₅- Aldehyd sind in technischen Gemisch zu etwa 40 - 50 % in alpha-Position verzweigt.

Beispiele weiterer geeigneter Alkohole sind lineare C₁₂₋₁₄ - Alkane mit einer Hydroxylgruppe in einer nicht endständigen Position entlang der Kette bzw. Gemische davon (z. B. Softanol® - Alkohole von Nippon Shokubai oder Tergitol®- Akohole von Dow).

v ist eine Zahl im Bereich von 3 bis 15. w hat den Wert 0. Es kann auf die vorstehenden Ausführungen zu x bei den kürzerkettigen Alkoholalkoxylaten verwiesen werden.

Die Erfindung betrifft auch ein Verfahren zur Herstellung von Alkoxylatgemischen, wie sie vorstehend beschrieben sind, bei dem Alkanole der allgemeinen Formel CₘH₂ₘ₊₁OH und 2-Propylheptanol mit der angegebenen Bedeutung für m mit Ethylenoxid unter Alkoxylierungsbedingungen umgesetzt werden und vor oder nach dem Alkoxylieren oder nach einem teilweisen Alkoxylieren miteinander gemischt werden.

Die Alkoxylierung kann beispielsweise unter Verwendung von alkalischen Katalysatoren wie Alkalihydroxiden, Alkalialkoholaten durchgeführt werden. Durch den Einsatz dieser Katalysatoren resultieren spezielle Eigenschaften, insbesondere der Verteilung des Alkoxylierungsgrades.

Die Alkoxylierung kann zudem unter Verwendung von Lewis-saurer Katalyse mit den daraus resultierenden speziellen Eigenschaften durchgeführt werden, insbesondere in Gegenwart von BF₃ x H₃PO₄, BF₃ Dietherat, BF₃, SbCl₅, SnCl₄ x 2 H₂O, Hydrotalcit. Geeignet als Katalysator sind auch Doppelmetallcyanid (DMC) Verbindungen.

Dabei kann der überschüssige Alkohol abdestilliert werden, oder das Alkoxylat kann durch einen Zwei-Stufen-Prozess gewonnen werden.

Vorzugsweise wird die Alkoxylierung durch starke Basen katalysiert, die zweckmäßigerweise in Form eines Alkalihydroxids oder Erdalkalihydroxids, in der Regel in einer Menge von 0,1 bis 1 Gew.-%, bezogen auf die Menge des Alkanols R²-OH, zugesetzt werden, (Vergl. G.Gee et al., J. Chem. Soc. (1961), S. 1345; B. Wojtech, Makromol. Chem. 66, (1966), S.180).

Auch eine saure Katalyse der Additionsreaktion ist möglich. Neben Bronstedsäuren eignen sich auch Lewissäuren, wie AlCl₃ oder BF₃. (Vergl. P.H.Plesch, The Chemistry of Cationic Polymerization, Pergamon Press, New York (1963)).

Als DMC-Verbindung können prinzipiell alle dem Fachmann bekannten geeigneten Verbindungen verwendet werden.

Als Katalysator geeignete DMC-Verbindungen sind beispielsweise in der WO 99/16775 und der DE-A-10117273 beschrieben. Insbesondere sind für die Alkoxylierung Doppelmetallcyanid-Verbindung der allgemeinen Formel I als Katalysator geeignet:

M¹ₐ[_{M}²(CN)_{b}(A)_{c}]_{d} fM¹_{g}Xₙ · h(H₂O) eL · kP (I),

in der
- M¹ mindestens ein Metallion, ausgewählt aus der Gruppe bestehend aus Zn²⁺ > Fe²⁺, > Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺ Sr²⁺, W⁴⁺, W⁶⁺, Cr²⁺, Cr³⁺, Cd²⁺ Hg²⁺, Pd²⁺, Pt²⁺, V²⁺, Mg²⁺, Ca²⁺, Ba²⁺, C²⁺, La³⁺, Ce³⁺, Ce⁴⁺, Eu³⁺, Ti³⁺, Ti⁴⁺, Ag⁺, Rh²⁺ Rh³⁺, Ru²⁺, Ru³⁺ ist,
- M² mindestens ein Metallion, ausgewählt aus der Gruppe bestehend aus Fe²⁺ Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺ ist,
- A und X unabhängig voneinander ein Anion, ausgewählt aus der Gruppe, bestehend aus Halogenid, Hydroxid, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,
- L ein mit Wasser mischbarer Ligand ist, ausgewählt aus der Gruppe, bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,
- k eine gebrochene oder ganze Zahl größer oder gleich Null ist, und
- P ein organischer Zusatzstoff ist,
- a, b, c, d, g und n so ausgewählt sind, dass die Elektroneutralität der Verbindung (I) gewährleistet ist, wobei c = 0 sein kann,
- e die Anzahl der Ligandenmoleküle eine gebrochenen oder ganze Zahl größer 0 oder 0 ist,
- f, h und m unabhängig voneinander eine gebrochene oder ganze Zahl größer 0 oder 0 sind.

Als organische Zusatzstoffe P sind zu nennen: Polyether, Polyester, Polycarbonate, Polyalkylenglykolsorbitanester, Polyakylenglykolglycidylether, Polyacrylamid, Poly(acrylamid-co-acrylsäure), Polyacrylsäure, Poly(acrylamid-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly(acrylsäure-co-styrol), Oxazolinpolymere, Polyalkylenimine, Maleinsäure- und Maleinsäureanhydridcopolymere, Hydroxyethylcellulose, Polyacetate, ionische oberflächen- und grenzflächenaktive Verbindungen, Gallensäure oder deren Salze, Ester oder Amide, Carbonsäureester mehrwertiger Alkohole und Glycoside.

Diese Katalysatoren können kristallin oder amorph sein. Für den Fall, dass k gleich null ist, sind kristalline Doppelmetallcyanid-Verbindungen bevorzugt. Im Fall, dass k größer null ist, sind sowohl kristalline, teilkristalline, als auch substantiell amorphe Katalysatoren bevorzugt.

Von den modifizierten Katalysatoren gibt es verschiedene bevorzugte Ausführungsformen. Eine bevorzugte Ausführungsform sind Katalysatoren der Formel (I), bei denen k größer null ist. Der bevorzugte Katalysator enthält dann mindestens eine Doppelmetallcyanid-Verbindung, mindestens einen organischen Liganden und mindestens einen organischen Zusatzstoff P.

Bei einer anderen bevorzugten Ausführungsform ist k gleich null, optional ist e auch gleich null und X ist ausschließlich ein Carboxylat, bevorzugt Formiat, Acetat und Propionat. Derartige Katalysatoren sind in der WO 99/16775 beschrieben. Bei dieser Ausführungsform sind kristalline Doppelmetallcyanid-Katalysatoren bevorzugt. Ferner bevorzugt sind Doppelmetallcyanid-Katalysatoren, wie in der WO 00/74845 beschrieben, die kristallin und plättchenförmig sind.

Die Herstellung der modifizierten Katalysatoren erfolgt durch Vereinigung einer Metallsalz-Lösung mit einer Cyanometallat-Lösung, die optional sowohl einen organischen Liganden L als auch einen organischen Zusatzstoff P enthalten können. Anschließend werden der organische Ligand und optional der organische Zusatzstoff zugegeben. Bei einer bevorzugten Ausführungsform der Katalysatorherstellung wird zunächst eine inaktive Doppelmetallcyanid-Phase hergestellt und diese anschließend durch Umkristallisation in eine aktive Doppelmetallcyanidphase überführt, wie in der PCT/EP01/01893 beschrieben.

Bei einer anderen bevorzugten Ausführungsform der Katalysatoren sind f, e und k ungleich Null. Dabei handelt es sich um Doppelmetallcyanid-Katalysatoren, die einen mit Wasser mischbaren organischen Ligand (im allgemeinen in Mengen von 0,5 bis 30 Gew.%) und einen organischen Zusatzstoff (im allgemeinen in Mengen von 5 bis 80 Gew.%) enthalten wie in der WO 98/06312 beschrieben. Die Katalysatoren können entweder unter starkem Rühren (24000U/Min mit Turrax) oder unter Rühren hergestellt werden wie in der US 5,158,922 beschrieben.

Insbesondere als Katalysator geeignet sind für die Alkoxylierung Doppelmetallcyanid-Verbindungen, die Zink, Kobalt oder Eisen oder zwei davon enthalten. Besonders geeignet ist beispielsweise Berliner Blau.

Bevorzugt werden kristalline DMC-Verbindungen eingesetzt. In einer bevorzugten Ausführungsform wird eine kristalline DMC-Verbindung vom Zn-Co-Typ als Katalysator verwendet, der als weitere Metallsalzkomponente Zinkacetat enthält. Derartige Verbindungen kristallisieren in monokliner Struktur und weisen einen plättchenförmigen Habitus auf. Derartige Verbindungen werden beispielsweise in der WO 00/74845 oder der PCT/EP01/01893 beschrieben.

Als Katalysator geeignete DMC-Verbindungen können prinzipiell auf alle dem Fachmann bekannten Arten hergestellt werden. Beispielsweise können die DMC-Verbindungen durch direkt Fällung, "incipient wetness"-Methode, durch Herstellung einer Precursor-Phase und anschließende Umkristallisation hergestellt werden.

Die DMC-Verbindungen können als Pulver, Paste oder Suspension eingesetzt werden oder zu einem Formkörper verformt werden, in Formkörpern, Schäume oder ähnliches eingebracht werden oder auf Formkörper, Schäume oder ähnliches aufgebracht werden.

Die zur Alkoxylierung eingesetzte Katalysator-Konzentration bezogen auf das Endmengengerüst ist typischerweise kleiner als 2000 ppm, bevorzugt kleiner als 1000 ppm, insbesondere kleiner als 500 ppm, besonders bevorzugt kleiner als 100 ppm, beispielsweise kleiner als 50 ppm.

Die Additionsreaktion wird bei Temperaturen von etwa 90 bis etwa 240°C, vorzugsweise von 120 bis 180°C, im geschlossenen Gefäß ausgeführt. Das Alkylenoxid oder die Mischung verschiedener Alkylenoxide wird der Mischung aus erfindungsgemäßem Alkanolgemisch und Alkali unter dem bei der gewählten Reaktionstemperatur herrschenden Dampfdruck des Alkylenoxidgemisches zugeführt. Gewünschtenfalls kann das Alkylenoxid mit bis zu etwa 30 bis 60 % mit einem Inertgas verdünnt werden. Dadurch wird eine zusätzliche Sicherheit gegen explosionsartige Polyaddition des Alkylenoxids gegeben.

Wird ein Alkylenoxidgemisch eingesetzt, so werden Polyetherketten gebildet in denen die verschiedenen Alkylenoxidbausteine praktisch statistisch verteilt sind. Variationen in der Verteilung der Bausteine längs der Polyetherkette ergeben sich aufgrund unterschiedlicher Reaktionsgeschwindigkeiten der Komponenten und können auch willkürlich durch kontinuierliche Zufuhr einer Alkylenoxidmischung programmgesteuerter Zusammensetzung erreicht werden. Werden die verschiedenen Alkylenoxide nacheinander zur Reaktion gebracht so erhält man Polyetherketten, mit blockartiger Verteilung der Alkylenoxid-Bausteine.

Die Länge der Polyetherketten schwankt innerhalb des Reaktionsprodukts statistisch um einen Mittelwert, der im wesentlichen dem sich aus der Zusatzmenge ergebenden stöchiometrischen Wert entspricht.

Die erfindungsgemäßen Alkoxylatgemische werden vorzugsweise in Wasch- oder Reinigungsmitteln eingesetzt, in denen sie zu einer Verbesserung der Wascheigenschaften führen. Die Erfindung betrifft auch ein Wasch- oder Reinigungsmittel, enthaltend ein wie vorstehend beschriebenes Alkoxylatgemisch. Das Alkoxylatgemisch wird dabei üblicherweise in einer Menge von 0,01 bis 80 Gew.%, vorzugsweise in einer Menge von 0,01 bis 50 Gew.-%, bezogen auf das Wasch- oder Reinigungsmittel, eingesetzt. Das Wasch- oder Reinigungsmittel wird vorzugsweise zum Waschen oder Reinigen von Textilien verwendet.

Der Mindestanteil der erfindungsgemäßen Alkoxylatgemische am Gesamtgewicht der erfindungsgemäßen Waschmittel wird so bemessen, dass sich eine signifikante Wirkung dieses Zusatzes zeigt. In der Regel wird eine gute Waschwirkung, insbesondere eine sehr gute Primärwaschwirkung der erfindungsgemäßen Waschmittel erreicht, wenn der Anteil der Gemische in dem erfindungsgemäßen Waschmittel, bezogen auf das Gesamtgewicht des Mittels, 0,01 bis 50 Gew.-%, vorzugsweise 0,1 bis 40 Gew.-%, insbesondere 0,5 bis 30 Gew.-% beträgt.

Waschmittel im Sinne dieser Erfindung dienen in der Regel zum Waschen von mehr oder weniger flexiblen Materialien,vorzugsweise solchen, die natürliche, synthetische oder halbsynthetische Fasermaterialien enthalten oder daraus bestehen und die demzufolge in der Regel zumindest teilweise einen textilen Charakter aufweisen. Die faserhaltigen oder aus Fasern bestehenden Materialien können prinzipiell in jeder im Gebrauch oder der Herstellung und Verarbeitung vorkommenden Form vorliegen. Beispielsweise können Fasern ungeordnet in Form von Flocke oder Haufwerk, geordnet in Form von Fäden, Garnen, Zwirnen, oder in Form von Flächengebilden wie Vliesen, Lodenstoffen oder Filz, Geweben, Gewirken in allen denkbaren Bindungsarten vorliegen.

Es kann sich um Rohfasern oder um Fasern in beliebigen Verarbeitungsstadien handeln und es können natürliche Eiweiß- oder Zellulosefasern wie Wolle, Seide, Baumwolle, Sisal, Hanf, Kokosfasern oder Synthesefasern wie beispielsweise Polyester-, Polyamid- oder Polyacrylnitrilfasern sein.

Die erfindungsgemäßen Waschmittel können auch zur Reinigung von faserhaltigen Materialien, wie z. B. rückenbeschichteten Teppichen mit geschnittenem oder ungeschnittenem Flor dienen.

Die Zusammensetzungen der Waschmittel werden vorzugsweise den verschiedenen Zwecken angepasst, wie es dem Fachmann aus dem Stand der Technik geläufig ist. Hierzu können den erfindungsgemäßen Waschmitteln alle zweckentsprechenden aus dem Stand der Technik bekannten Hilfs- und Zusatzstoffe zugefügt werden.

In Waschmitteln können neben den erfindungsgemäßen Gemischen beispielsweise vorliegen:
- Builder und Cobuilder, wie Polyphosphate, Zeolithe, Polycarboxylate, Phosphonate oder Komplexbildner
- ionische Tenside, wie Alkoholsulfate/-ethersulfate, Alkylbenzolsulfonate, α-Olefinsulfonate und andere Alkoholsulfate/-ethersulfate
- andere nichtionische Tenside, wie Alkylaminalkoxylate, Alkylpolyglucoside
- optische Aufheller,
- Farbübertragungsinhibitoren, wie Polyvinylpyrrolidon der Molmassen 8.000 bis 70.000, Vinylimidazol/Vinylpyrrolidon-Copolymere mit einem Molverhältnis der Monomeren von 1:10 bis 2:1 und Molmassen von 8.000 bis 70.000 sowie Poly-4-vinylpyridin-N-oxide mit Molmassen von 8.000 bis 70.000.
- Stellmittel, wie Natriumsulfat oder Magnesiumsulfat
- Soil Release-Mittel
- Inkrustationsinhibitoren
- Bleichsysteme, enthaltend Bleichmittel, wie Perborat, Percarbonat und Bleichakitvatoren, wie Tetraacetylethylendiamin sowie Bleichstabilisatoren
- Parfum/-öle
- Schaumdämpfer, wie Silikonöle
- Enzyme, wie Amylasen, Lipasen, Cellulasen, Proteasen
- Alkalispender, wie lösliche Alkalisilikate, z. B. Pentanatriummethasilikat, Nariumcarbonat.

In flüssigen Waschmitteln können beispielsweise zusätzlich Lösungsmittel, wie Ethanol, Isopropanol, 1,2-Propylenglycol, Butylglycol usw. eingesetzt werden.

In tablettenförmigen Waschmitteln können zusätzlich Tablettierhilfsmittel, wie Polyethylenglycole mit Molmassen von mehr als 1000 g/mol, Polymerdispersionen sowie Tablettensprengmittel, wie Cellulosederivate, vernetztes Polyvinylpyrrolidon, vernetzte Polyacrylate oder Kombinationen aus Säuren, wie Zitronensäure und Natriumbicarbonat eingesetzt werden. Eine detailliertere Ausführung möglicher Inhaltsstoffe wird nachfolgend gegeben.

In manchen Fällen kann es zweckmäßig sein, die erfindungsgemäß eingesetzten Gemische mit anderen nichtionischen Tensiden, wie Alkylaminalkoxilaten, Alkylamidalkoxilaten, Alkylpolyglucosiden, oder mit ionischen, vorzugsweise anionischen, Tensiden, wie z. B. Alkoholsulfat/-ethersulfaten, Alkylbenzolsulfonaten, α-Olefinsulfonaten, Sulfosuccinaten, oder mit amphoteren Tensiden, wie z. B. Alkylaminoxiden, oder Betainen zu kombinieren.

Im Folgenden werden Beispiele für zur Kombination geeignete Tenside unterschiedlicher Natur genannt:
Eine Klasse geeigneter nichtionischer Tenside sind Alkylphenolalkoxylate wie Alkylphenolethoxylate mit C₆ bis C₁₄-Alkylketten und 5 bis 30 Mol Alkylenoxideinheiten.
Eine andere Klasse nichtionischer Tenside sind Alkylpolyglucoside mit 6 bis 22, vorzugsweise 10 bis 18 Kohlenstoffatomen in der Alkylkette. Diese Verbindungen enthalten meist 1 bis 20, vorzugsweise 1,1 bis 5 Glucosideinheiten.
Eine andere Klasse nichtionischer Tenside sind N-Alkylglucamide der allgemeinen Strukturen 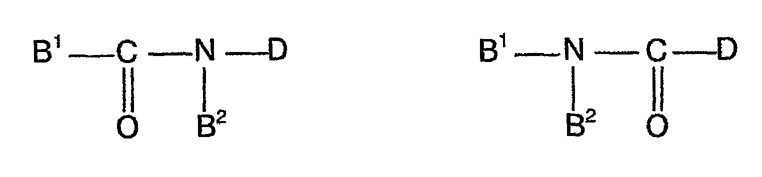 wobei B¹ ein C₆- bis C₂₂-Alkyl, B² Wasserstoff oder C₁- bis C₄-Alkyl und D ein Polyhydroxyalkyl-Rest mit 5 bis 12 C-Atomen und mindestens 3 Hydroxygruppen ist. Vorzugsweise steht B¹ für C₁₀- bis C₁₈-Alkyl, B² für CH₃ und D für einen C₅- oder C₆-Rest. Beispielsweise erhält man derartige Verbindungen durch die Acylierung von reduzierend aminierten Zuckern mit Säurechloriden von C₁₀- bis C₁₈-Carbonsäuren.

Weitere in Betracht kommende nichtionische Tenside sind die aus der WO-A 95/11225 bekannten endgruppenverschlossenen Fettsäureamidalkoxylate der allgemeinen Formel

R¹-CO-NH- (CH₂)_{y}-O- (A¹O)ₓ-R²

in der
- R¹: einen C₅- bis C₂₁-Alkyl- oder Alkenylrest bezeichnet,
- R²: eine C₁- bis C₄-Alkylgruppe bedeutet,
- A¹: für C₂- bis C₄-Alkylen steht,
- y: die Zahl 2 oder 3 bezeichnet und
- x: einen Wert von 1 bis 6 hat.

Beispiele für solche Verbindungen sind die Umsetzungsprodukte von n-Butyltriglykolamin der Formel H₂N-(CH₂-CH₂-O)₃-C₄H₉ mit Dodecansäuremethylester oder die Reaktionsprodukte von Ethyltetraglykolamin der Formel H₂N-(CH₂-CH₂-O)₄-C₂H₅ mit einem handelsüblichen Gemisch von gesättigten C₈- bis C₁₈-Fettsäuremethylestern.

Weiterhin eignen sich als nichtionische Tenside noch Blockcopolymere aus Ethylenoxid, Propylenoxid und/oder Butylenoxid (Pluronic®- und Tetronic®-Marken der BASF), Polyhydroxy- oder Polyalkoxyfettsäurederivate wie Polyhydroxyfettsäureamide, N-Alkoxy- oder N-Aryloxypolyhydroxyfettsäureamide, Fettsäureamidethoxylate, insbesondere endgruppenverschlossene, sowie Fettsäurealkanolamidalkoxylate.

Die zusätzlichen nichtionischen Tenside("Niotenside") liegen in den erfindungsgemäßen Waschmitteln vorzugsweise in einer Menge von 0,01 bis 30 Gew.-%, insbesondere 0,1 bis 25 Gew.-%, vor allem 0,5 bis 20 Gew.-%, vor.

Man kann zusätzlich einzelne nichtionische Tenside oder eine Kombination unterschiedlicher Niotenside einsetzen. Es können nichtionische Tenside aus nur einer

Klasse zum Einsatz gelangen, insbesondere nur alkoxylierte C₈- bis C₂₂-Alkohole, man kann aber auch Tensidmischungen aus verschiedenen Klassen verwenden.

Geeignete anionische Tenside sind beispielsweise Fettalkoholsulfate von Fettalkoholen mit 8 bis 22, vorzugsweise 10 bis 18 Kohlenstoffatomen, C₁₂-C₁₈-Alkoholsulfate, Laurylsulfat, Cetylsulfat, Myristylsulfat, Palmitylsulfat, Stearylsulfat und Talgfettalkoholsulfat.

Weitere geeignete anionische Tenside sind sulfatierte ethoxylierte C₈- bis C₂₂-Alkohole (Alkylethersulfate) bzw. deren lösliche Salze. Verbindungen dieser Art werden beispielsweise dadurch hergestellt, dass man zunächst einen C₈- bis C₂₂-, vorzugsweise einen C₁₀- bis C₁₈-Alkohol z. B. einen Fettalkohol, alkoxyliert und das Alkoxylierungsprodukt anschließend sulfatiert. Für die Alkoxylierung verwendet man vorzugsweise Ethylenoxid, wobei man pro Mol Alkohol 1 bis 50, vorzugsweise 1 bis 20 Mol Ethylenoxid einsetzt. Die Alkoxylierung der Alkohole kann jedoch auch mit Propylenoxid allein und gegebenenfalls Butylenoxid durchgeführt werden. Geeignet sind außerdem solche alkoxylierte C₈- bis C₂₂-Alkohole, die Ethylenoxid und Propylenoxid oder Ethylenoxid und Butylenoxid oder Ethylenoxid und Propylenoxid und Butylenoxid enthalten. Die alkoxylierten C₈- bis C₂₂-Alkohole können die Ethylenoxid-, Propylenoxidund Butylenoxideinheiten in Form von Blöcken oder in statistischer Verteilung enthalten. Je nach Art des Alkoxylierungskatalysators kann man Alkylethersulfate mit breiter oder enger Alkylenoxid-Homologen-Verteilung erhalten.

Weitere geeignete anionische Tenside sind Alkansulfonate wie C₈- bis C₂₄-, vorzugsweise C₁₀- bis C₁₈-Akansulfonate sowie Seifen wie beispielsweise die Na- und K-Salze von gesättigten und/oder ungesättigten C₈- bis C₂₄-Carbonsäuren.

Weitere geeignete anionische Tenside sind lineare C₈- bis C₂₀-Alkylbenzolsulfonate ("LAS"), vorzugsweise lineare C₉- bis C₁₃-Alkylbenzolsulfonate und -Alkyltoluolsulfonate.

Weiterhin eignen sich als anionische Tenside noch C₈- bis C₂₄-Olefinsulfonate und -disulfonate, welche auch Gemische aus Alken- und Hydroxyalkansulfonaten bzw. -disulfonate darstellen können, Alkylestersulfonate, sulfonierte Polycarbonsäuren, Alkylglycerinsulfonate, Fettsäureglycerinestersulfonate, Alkylphenolpolyglykolethersulfate, Paraffinsulfonate mit ca. 20 bis ca. 50 C-Atomen (basierend auf aus natürlichen Quellen gewonnenem Paraffin oder Paraffingemischen), Alkylphosphate, Acylisethionate, Acyltaurate, Acylmethyltaurate, Alkylbernsteinsäuren, Alkenylbernsteinsäuren oder deren Halbester oder Halbamide, Alkylsulfobernsteinsäuren oder deren Amide, Mono- und Diester von Sulfobernsteinsäuren, Acylsarkosinate, sulfatierte Alkylpolyglucoside, Alkylpolyglykolcarboxylate sowie Hydroxyalkylsarkosinate.

Die anionischen Tenside werden dem Waschmittel vorzugsweise in Form von Salzen zugegeben. Geeignete Kationen in diesen Salzen sind Alkalimetallionen wie Natrium, Kalium und Lithium und Ammoniumsalze wie z. B. Hydroxyethylammonium-, Di(hydroxyethyl)ammonium- und Tri(hydroxyethyl)ammoniumsalze.

Die anionischen Tenside liegen in den erfindungsgemäßen Waschmitteln vorzugsweise in einer Menge von bis zu 30 Gew.-%, beispielsweise von 0,1 bis 30 Gew.-%, vor allem 1 bis 25 Gew.%, insbesondere 3 bis 20 Gew.-% vor. Werden C₉- bis C₂₀-linear-Alkylbenzolsulfonate (LAS) mitverwendet, kommen diese üblicherweise in einer Menge bis zu 15 Gew.-%, insbesondere bis zu 10 Gew.-%, zum Einsatz.

Man kann einzelne anionische Tenside oder eine Kombination unterschiedlicher Aniontenside einsetzen. Es können anionische Tenside aus nur einer Klasse zum Einsatz gelangen, beispielsweise nur Fettalkoholsulfate oder nur Alkylbenzolsulfonate, man kann aber auch Tensidmischungen aus verschiedenen Klassen verwenden, z. B. eine Mischung aus Fettalkoholsulfaten und Alkylbenzolsulfonaten.

Ferner können die erfindungsgemäß einzusetzenden Tensidgemische mit kationischen Tensiden, üblicherweise in einer Menge bis 25 Gew.-%, vorzugsweise 1 bis 15 Gew.-%, beispielsweise C₈-bis C₁₆-Dialkyldimethylammoniumsalzen, Dialkoxydimethylammoniumsalzen oder Imidazoliniumsalzen mit langkettigem Alkylrest; und/oder mit amphoteren Tensiden, üblicherweise in einer Menge bis 15 Gew.-%, vorzugsweise 1 bis 10 Gew.-%, beispielsweise Derivaten von sekundären oder tertiären Aminen wie z. B. C₆-C₁₈-Alkylbetainen oder C₆-C₁₅-Alkylsulfobetainen oder Alkylamidobetainen oder Aminoxiden wie Alkyldimethylaminoxiden kombiniert werden.

Ferner können kationische Tenside eingesetzt werden, wie sie in der WO 99/19435 beschrieben sind.

In der Regel werden die erfindungsgemäß einzusetzenden Gemische mit Buildern (Sequestrierungsmitteln) wie z. B. Polyphosphaten, Polycarboxilaten, Phosphonaten, Komplexbildnern, z. B. Methylglycindiessigsäure und deren Salze, Nitrilotriessigsäure und deren Salze, Ethylendiamintetraessigsäure und deren Salze sowie gegebenenfalls mit Co-Buildern kombiniert.

Einzelne zur Kombination mit den erfindungsgemäß einzusetzenden Gemischen gut geeignete Buildersubstanzen seien im Folgenden aufgezählt:
Geeignete anorganische Builder sind vor allem kristalline oder amorphe Alumosilicate mit ionenaustauschenden Eigenschaften wie insbesondere Zeolithe. Verschiedene Typen von Zeolithen sind geeignet, insbesondere Zeolithe A, X, B, P, MAP und HS in ihrer Na-Form oder in Formen, in denen Na teilweise gegen andere Kationen wie Li, K, Ca, Mg oder Ammonium ausgetauscht ist. Geeignete Zeolithe sind beispielsweise beschrieben in der US-A-4604224.

Als Builder geeignete kristalline Silicate sind beispielsweise Disilicate oder Schichtsilicate, z. B. δ-Na₂Si₂O₅ oder β-Na₂Si₂O₅. Die Silicate können in Form ihrer Alkalimetall-, Erdalkalimetall- oder Ammoniumsalze eingesetzt werden, vorzugsweise als Na-, Li- und Mg-Silicate.Amorphe Silicate wie beispielsweise Natriummetasilicat, welches eine polymere Struktur aufweist, oder amorphes Disilicat sind ebenfalls verwendbar.

Geeignete anorganische Buildersubstanzen auf Carbonat-Basis sind Carbonate und Hydrogencarbonate. Diese können in Form ihrer Alkalimetall-, Erdalkalimetall- oder Ammoniumsalze eingesetzt werden. Vorzugsweise werden Na-, Li- und Mg-Carbonate bzw. - Hydrogencarbonate, insbesondere Natriumcarbonat und/oder Natriumhydrogencarbonat, eingesetzt.

Übliche, als anorganische Builder eingesetzte Phosphate sind Alkaliorthophosphate,und/oder -Polyphosphate wie z. B. Pentanatriumtriphosphat.

Die genannten Builder-Komponenten können einzeln oder in Mischungen untereinander eingesetzt werden.

Ferner ist es in vielen Fällen zweckmäßig den erfindungsgemäßen Waschmitteln Co-Builder zuzufügen. Beispiele für geeignete Substanzen sind im Folgenden aufgelistet:
In einer bevorzugten Ausführungsform enthalten die erfindungsgemäßen Waschmittel zusätzlich zu den anorganischen Buildern 0,05 bis 20 Gew.-%, insbesondere 1 bis 10 Gew.-% organische Cobuilder in Form von niedermolekularen, oligomeren oder polymeren Carbonsäuren, insbesondere Polycarbonsäuren, oder Phosphonsäuren oder deren Salzen, insbesondere Na- oder K-salzen.

Als organische Cobuilder geeignete niedermolekulare Carbonsäuren oder Phosphonsäuren sind beispielsweise:
Phosphonsäuren wie z. B. 1-Hydroxyethan-1,1-diphosphonsäure, Aminotris(methylenphosphonsäure), Ethylendiamin-tetra(methylenphosphonsäure) Hexamethylendiamin-tetra (methylenphosphonsäure) und Diethylentriaminpenta(methylenphosphonsäure);
C₄-bis C₂₀-Di-, -Tri- und -Tetracarbonsäuren wie z. B. Bernsteinsäure, Propantricarbonsäure, Butantetracarbonsäure, Cyclopentantetracarbonsäure und Alkyl- und Alkenylbernsteinsäuren mit C₂- bis C₁₆-Alkyl- bzw. -Alkenyl-Resten;
C₄- bis C₂₀-Hydroxycarbonsäuren wie z. B. Äpfelsäure, Weinsäure,
Gluconsäure, Glutarsäure, Citronensäure, Lactobionsäure und Saccharosemono-, di- und tricarbonsäure;
Aminopolycarbonsäuren wie z. B. Nitrilotriessigsäure, β-Alanindiessigsäure, Ethylendiamintetraessigsäure, Serindiessigsäure, Isoserindiessigsäure, Alkylethylendiamintriacetate, N,N-bis(Carboxymethyl)glutaminsäure, Ethylendiamindibernsteinsäure und N-(2-Hydroxyethyl)iminodiessigsäure, Methyl- und Ethylglycindiessigsäure.

Als organische Cobuilder geeignete oligomere oder polymere Carbonsäuren sind beispielsweise:
Oligomaleinsäuren, wie sie beispielsweise in EP-A 451508 und EP-A 396303 beschrieben sind;
Co- und Terpolymere ungesättigter C₄- bis C₈-Dicarbonsäuren, wobei als Comonomere monoethylenisch ungesättigte Monomere aus der unten angegebenen Gruppe (i) in Mengen von bis zu 95 Gew.-%, aus der Gruppe (ii) in Mengen von bis zu 60 Gew.-% und aus der Gruppe (iii) in Mengen von bis zu 20 Gew.-% einpolymerisiert sein können.

Als ungesättigte C₄- bis C₈-Dicarbonsäuren sind hierbei beispielsweise Maleinsäure, Fumarsäure, Itaconsäure und Citraconsäure geeignet. Bevorzugt wird Maleinsäure.

Die Gruppe (i) umfasst monoethylenisch ungesättigte C₃-C₈-Monocarbonsäuren wie z. B. Acrylsäure, Methacrylsäure, Crotonsäure und Vinylessigsäure. Bevorzugt werden aus der Gruppe (i) Acrylsäure und Methacrylsäure eingesetzt.

Die Gruppe (ii) umfasst monoethylenisch ungesättigte C₂- bis C₂₂-Olefine, Vinylalkylether mit C₁- bis C₈-Alkylgruppen, Styrol, Vinylester von C₁- bis C₈-Carbonsäuren, (Meth)acrylamid und Vinylpyrrolidon. Bevorzugt werden aus der Gruppe (ii) C₂- bis C₆-Olefine, Vinylalkylether mit C₁- bis C₄-Alkylgruppen, Vinylacetat und Vinylpropionat eingesetzt.

Falls die Polymeren der Gruppe (ii) Vinylester einpolymerisiert enthalten, können diese auch teilweise oder vollständig zu Vinylalkohol-Struktureinheiten hydrolysiert vorliegen. Geeignete Co- und Terpolymere sind beispielsweise aus US-A 3887806 sowie DE-A 4313909 bekannt.

Die Gruppe (iii) umfasst (Meth)acrylester von C₁- bis C₈-Alkoholen, (Meth)acrylnitril, (Meth)acrylamide von C₁- bis C₈-Aminen, N-Vinylformamid und N-Vinylimidazol.

Als organische Cobuilder eignen sich auch Homopolymere der monoethylenisch ungesättigten C₃-C₈-Monocarbonsäuren wie z. B. Acrylsäure, Methacrylsäure, Crotonsäure und Vinylessigsäure, insbesondere der Acrylsäure und Methacrylsäure,
Copolymere von Dicarbonsäuren, wie z. B. Copolymere von Maleinsäure und Acrylsäure im Gewichtsverhältnis 10:90 bis 95:5, besonders bevorzugt solche im Gewichtsverhältnis 30:70 bis 90:10 mit Molmassen von 1000 bis 150000;
Terpolymere aus Maleinsäure, Acrylsäure und einem Vinylester einer C₁-C₃-Carbonsäure im Gewichtsverhältnis 10 (Maleinsäure) :90 (Acrylsäure + Vinylester) bis 95 (Maleinsäure) :10 (Acrylsäure + Vinylester), wobei das Gew.-Verhältnis von Acrylsäure zum Vinylester im Bereich von 30:70 bis 70:30 variieren kann;

Copolymere von Maleinsäure mit C₂-C₈-Olefinen im Molverhältnis 40:60 bis 80:20, wobei Copolymere von Maleinsäure mit Ethylen, Propylen oder Isobuten im Molverhältnis 50:50 besonders bevorzugt sind.

Pfropfpolymere ungesättigter Carbonsäuren auf niedermolekulare Kohlenhydrate oder hydrierte Kohlenhydrate, vgl. US-A 5227446, DE-A 4415623 und DE-A 4313909, eignen sich ebenfalls als organische Cobuilder.

Geeignete ungesättigte Carbonsäuren sind hierbei beispielsweise Maleinsäure, Fumarsäure, Itaconsäure, Citraconsäure, Acrylsäure, Methacrylsäure, Crotonsäure und Vinylessigsäure sowie Mischungen aus Acrylsäure und Maleinsäure, die in Mengen von 40 bis 95 Gew.%, bezogen auf die zu pfropfende Komponente, aufgepfropft werden.

Zur Modifizierung können zusätzlich bis zu 30 Gew.-%, bezogen auf die zu pfropfende Komponente, weitere monoethylenisch ungesättigte Monomere einpolymerisiert vorliegen. Geeignete modifizierende Monomere sind die oben genannten Monomere der Gruppen (ii) und (iii).

Als Pfropfgrundlage sind abgebaute Polysaccharide wie z. B. sauer oder enzymatisch abgebaute Stärken, Inuline oder Zellulose, Eiweißhydrolysate und reduzierte (hydrierte oder hydrierend aminierte) abgebaute Polysaccharide wie z. B. Mannit, Sorbit, Aminosorbit und N-Alkylglucamin geeignet sowie auch Polyalkylenglycole mit Molmassen mit bis zu M_{w} = 5000 wie z. B. Polyethylenglycole, Ethylenoxid/Propylenoxid- bzw. Ethylenoxid/Butylenoxid bzw. Ethylenoxid/Propylenoxid/Butylenoxid-Blockcopolymere und alkoxylierte ein- oder mehrwertige C₁- bis C₂₂-Alkohole.(vgl. US-A-5756456)

Als organische Cobuilder geeignete Polyglyoxylsäuren sind beispielsweise beschrieben in EP-B-001004, US-A-5399286, DE-A-4106355 und EP-A-656914. Die Endgruppen der Polyglyoxylsäuren können unterschiedliche Strukturen aufweisen.

Als organische Cobuilder geeignete Polyamidocarbonsäuren und modifizierte Polyamidocarbonsäuren sind beispielsweise bekannt aus EP-A-454126, EP-B-511037, WO-A-94/01486 und EP-A-581452.

Als organische Cobuilder verwendet man insbesondere auch Polyasparaginsäuren oder Cokondensate der Asparaginsäure mit weiteren Aminosäuren, C₄- bis C₂₅-Mono- oder - Dicarbonsäuren und/oder C₄- bis C₂₅-Mono- oder -Diaminen. Besonders bevorzugt werden in phosphorhaltigen Säuren hergestellte, mit C₆- bis C₂₂-Mono- oder -Dicarbonsäuren bzw. mit C₆- bis C₂₂-Mono- oder -Diaminen modifizierte Polyasparaginsäuren eingesetzt.

Als organische Cobuilder eignen sich weiterhin Iminodibemsteinsäure, Oxydibernsteinsäure, Aminopolycarboxylate, Alkylpolyaminocarboxylate, Aminopolyalkylenphosphonate, Polyglutamate, hydrophob modifizierte Citronensäure wie z. B. Agaricinsäure, Poly-α-hydroxyacrylsäure, N-Acylethylendiamintriacetate wie Lauroylethylendiamintriacetat und Alkylamide der Ethylendiamintetraessigsäure wie EDTA-Talgamid.

Weiterhin können auch oxidierte Stärken als organische Cobuilder verwendet werden.

Weitere geeignete (Co)builder sind in WO 99/19435 beschrieben.

In einer weiteren bevorzugten Ausführungsform enthalten die erfindungsgemäßen Waschmittel zusätzlich, insbesondere zusätzlich zu den anorganischen Buildem, den anionischen Tensiden und/oder den nichtionischen Tensiden, 0,5 bis 20 Gew.-%, insbesondere 1 bis 10 Gew.-%, Glycin-N,N-diessigsäure-Derivate, wie sie in der WO 97/19159 beschrieben sind.

Häufig ist es auch zweckmäßig, den erfindungsgemäßen Waschmitteln Bleichsysteme, bestehend aus Bleichmitteln, wie z. B. Perborat, Percarbonat und gegebenenfalls Bleichaktivatoren, wie z. B. Tetraacetylethylendiamin, + Bleichstabilisatoren sowie gegebenenfalls Bleichkatalysatoren zuzusetzen.

In diesen Fällen enthalten die erfindungsgemäßen Waschmittel zusätzlich 0,5 bis 30 Gew.-%, insbesondere 5 bis 27 Gew.-%, vor allem 10 bis 23 Gew.-% Bleichmittel in Form von Percarbonsäuren, z. B. Diper-oxododecandicarbonsäure, Phthalimidopercapronsäure oder Monoperoxophthalsäure oder -terephthalsäure, Addukten von Wasserstoffperoxid an anorganische Salze, z. B. Natriumperborat-Monohydrat, Natriumperborat-Tetrahydrat, Natriumcarbonat-Perhydrat oder Natriumphosphat-Perhydrat, Addukten von Wasserstoffperoxid an organische Verbindungen, z. B. Harnstoff-Perhydrat, oder von anorganischen Peroxosalzen, z. B. Alkalimetallpersulfaten, oder -peroxodisulfaten, gegebenenfalls in Kombination mit 0 bis 15 Gew.-%, vorzugsweise 0,1 bis 15 Gew.-%, insbesondere 0,5 bis 8 Gew.-%, Bleichaktivatoren.

Als Bleichaktivatoren eignen sich:
- polyacylierte Zucker, z. B. Pentaacetylglucose;
- Acyloxybenzolsulfonsäuren und deren Alkali- und Erdalkaiimetallsalze, z. B. Natrium-p-nonanoyloxybenzolsulfonat oder Natrium-p-benzoyloxybenzolsulfonat;
- N,N-diacylierte und N,N,N',N'-tetraacylierte Amine, z. B. N,N,N',N'-Tetraacetylmethylendiamin und -ethylendiamin (TAED), N,N-Diacetylanilin, N,N-Diacetyl-p-toluidin oder 1,3-diacylierte Hydantoine wie 1,3-Diacetyl-5,5-dimethylhydantoin;
- N-Alkyl-N-sulfonylcarbonamide, z. B. N-Methyl-N-mesylacetamid oder N-Methyl-N-mesylbenzamid;
- N-acylierte cyclische Hydrazide, acylierte Triazole oder Urazole, z. B. Monoacetylmaleinsäurehydrazid;
- O,N,N-trisubstituierte Hydroxylamine, z. B. O-Benzoyl-N,N-succinylhydroxylamin, O-Acetyl-N,N-succinylhydroxylamin oder O,N,N-Triacetylhydroxylamin;
- N,N'-Diacylsulfurylamide, z. B. NN'-Dimethyl-NN'-diacetylsulfurylamid oder N,N'-Diethyl-N,N'-dipropionyisulfurylamid;
- acylierte Lactame wie beispielsweise Acetylcaprolactam, Octanoylcaprolactam, Benzoylcaprolactam oder Carbonylbiscaprolactam;
- Anthranilderivate wie z. B. 2-Methylanthranil oder 2-Phenylanthranil;
- Triacylcyanurate, z. B. Triacetylcyanurat oder Tribenzoylcyanurat;
- Oximester und Bisoximester wie z. B. O-Acetylacetonoxim oder Bisisopropyliminocarbonat;
- Carbonsäureanhydride, z. B. Essigsäureanhydrid, Benzoesäureanhydrid, m-Chlorbenzoesäureanhydrid oder Phthalsäureanhydrid;
- Enolester wie z. B. Isopropenylacetat;
- 1,3-Diacyl-4,5-diacyloxy-imidazoline, z. B. 1,3-Diacetyl-4,5-diacetoxyimidazolin;
- Tetraacetylglycoluril und Tetrapropionylglycoluril;
- diacylierte 2,5-Diketopiperazine, z. B. 1,4-Diacetyl-2,5-diketopiperazin;
- ammoniumsubstituierte Nitrile wie z. B. N-Methylmorpholiniumacetonitrilmethylsulfat;
- Acylierungsprodukte von Propylendiharnstoff und 2,2-Dimethylpropylendiharnstoff, z. B. Tetraacetylpropylendiharnstoff;
- α-Acyloxypolyacylmalonamide, z. B. α-Acetoxy-N,N'-diacetylmalonamid;
- Diacyl-dioxohexahydro-1,3,5-triazine, z. B. 1,5-Diacetyl-2,4-dioxohexahydro-1,3,5-triazin;
- Benz-(4H)1,3-oxazin-4-one mit Alkylresten, z. B. Methyl, oder aromatischen Resten z. B. Phenyl, in der 2-Position;
- kationische Nitrile, wie in DE-A-101 48 577 beschrieben.

Das beschriebene Bleichsystem aus Bleichmitteln und Bleichaktivatoren kann gegebenenfalls noch Bleichkatalysatoren enthalten. Geeignete Bleichkatalysatoren sind beispielsweise quaternierte Imine und Sulfonimine, die beispielsweise beschrieben sind in US-A 5 360 569 und EP-A 453 003. Besonders wirksame Bleichkatalysatoren sind Mangankomplexe, die beispielsweise in der WO-A 94/21777 beschrieben sind. Solche Verbindungen werden im Falle ihres Einsatzes in den Waschmitteln höchstens in Mengen bis 1,5 Gew.-%, insbesondere bis 0,5 % Gew.-%, im Falle von sehr aktiven Mangankomplexen in Mengen bis zu 0,1 Gew.-%, eingearbeitet. Weitere geeignete Bleichkatalysatoren sind in WO 99/19435 beschrieben.

Weitere einsetzbare Bleichsysteme auf Basis von Arylimidoperalkansäuren sind in EP-A-0 325 288 und EP-A-0 490 409 beschrieben.

### Bleichstabilisator

Dabei handelt es sich um Additive, die Schwermetallspuren absorbieren, binden oder komplexieren können. Beispiele für erfindungsgemäß verwendbare Zusätze mit bleichstabilisierender Wirkung sind polyanionische Verbindungen wie Polyphosphate, Polycarboxylate, Polyhydroxypolycarboxylate, lösliche Silikate als vollständig oder teilweise neutralisierte Alkali- oder Erdalkalisalze, insbesondere als neutrale Na- oder Mg-Salze, die relativ schwache Bleichstabilisatoren sind. Starke erfindungsgemäß verwendbare Bleichstabilisatoren sind beispielsweise Komplexbildner, wie Ethylendiamintetraacetat (EDTA), Nitrilotriessigsäure (NTA), Methylglycindiessigsäure (MGDA), β-Alanindiessigsäure (ADA), Ethylendiamin-N,N'-diesuccinat (EDDS) und Phosphonate wie Ethylendiamintetramethylenphosphonat, Diethylentriaminpentamethylenphosphonat oder Hydroxyethyliden-1,1-diphosphonsäure in Form der Säuren oder als teilweise oder vollständig neutralisierte Alkalimetallsalze. Vorzugsweise werden die Komplexbildner in Form ihrer Na-Salze eingesetzt.

Neben dem beschriebenen Bleichsystem aus Bleichmitteln, Bleichaktivatoren und gegebenenfalls Bleichkatalysatoren ist für die erfindungsgemäßen Waschmittel auch die Verwendung von Systemen mit enzymatischer Peroxidfreisetzung oder von photoaktivierten Bleichsystemen möglich, siehe z. B. US 4,033,718.

Für eine Reihe von Anwendungsfällen ist es zweckmäßig, wenn die erfindungsgemäßen Waschmittel Enzyme enthalten. Vorzugsweise in Waschmitteln eingesetzte Enzyme sind Proteasen, Amylasen, Lipasen und Cellulasen. Von den Enzymen werden vorzugsweise mengen von 0,1 bis 1,5 Gew.-%, insbesondere vorzugsweise 0,2 bis 1,0 Gew.-%, des konfektionierten Enzyms zugesetzt. Geeignete Proteasen sind z. B. Savinase und Esperase. Eine geeignete Lipase ist z. B. Lipolase. Eine geeignete Cellulase ist z. B. Celluzym. Auch die Verwendung von Peroxidasen zur Aktivierung des Bleichsystems ist möglich. Man kann einzelne Enzyme oder eine Kombination unterschiedlicher Enzyme einsetzen. Gegebenenfalls kann das erfindungsgemäße Waschmittel noch Enzymstabilisatoren, z. B. Calciumpropionat, Natriumformiat oder Borsäuren oder deren Salze, und/oder Oxidationsverhinderer enthalten.

Die Bestandteile von Waschmitteln sind dem Fachmann prinzipiell bekannt. Die obigen und die weiter unten folgenden Listen geeigneter Bestandteile geben nur einen exemplarischen Ausschnitt der bekannten geeigneten Bestandteile wieder.

Die erfindungsgemäßen Waschmittel können neben den bisher genannten Hauptkomponenten noch folgende weitere übliche Zusätze in den hierfür üblichen Mengen enthalten:
Bekannte Dispergiermittel, wie Naphthalinsulfonsäurekondensate oder Polycarboxilate, Schmutztragemittel, Soil release Agentien, wie Polyetherester, Inkrustationsinhibitoren, pH-regulierende Verbindungen wie Alkalien bzw. Alkalispender (NaOH, KOH, Pentanatriummetasilikat, Natriumcarbonat) oder Säuren (Salzsäure, Phosphorsäure, Amidoschwefelsäure, Citronensäure) Puffersysteme, wie Acetat oder Phosphatpuffer, Ionenaustauscher, Parfüm, Farbstoffe, Vergrauungsinhibitoren, optische (fluoreszierende) Aufheller, Farbübertragungsinhibitoren wie z. B. Polyvinylpyrrolidon, Biozide, wie Isothiazolinone oder 2-Bromo-2-nitro-1,3-propandiol, hydrotrope Verbindungen als Lösungsvermittler bzw. Solubilisatoren, wie Cumolsulfonate, Toluolsulfonate, kurzkettige Fettsäuren, Harnstoff, Alkohole oder Phosphorsäurealkyl/-arylester, Schaumregulatoren zur Stabilisierung oder Dämpfung des Schaums, z. B. Siliconöle, Haut- und Korrosionsschutzmittel, desinfizierende Verbindungen oder Systeme, wie z. B. solche die Chlor oder unterchlorige Säure freisetzen wie Dichlorisocyanurat oder die Iod enthalten, Verdickungsmittel und Stell- und Konfektionierungsmittel.

### Vergrauungsinhibitoren und Soil-Release-Polymere

Geeignete Soil-Release-Polymere und/oder Vergrauungsinhibitoren für Waschmittel sind beispielsweise:
Polyester aus Polyethylenoxiden mit Ethylenglycol und/oder Propylenglycol und aromatischen Dicarbonsäuren oder aromatischen und aliphatischen Dicarbonsäuren;
Polyester aus einseitig endgruppenverschlossenen Polyethylenoxiden mit zwei-und/oder mehrwertigen Alkoholen und Dicarbonsäure.

Derartige Polyester sind bekannt, beispielsweise aus US-A 3,557,039, GB-A 1 154 730, EP-A-185 427, EP-A-241 984, EP-A-241 985, EP-A- 272 033 und US-A 5,142,020.

Weitere geeignete Soil-Release-Polymere sind amphiphile Pfropf- oder Copolymere von Vinyl-und/oder Acrylestern auf Polyalkylenoxide (vgl. US-A 4,746,456, US-A 4,846,995, DE-A-37 11 299, US-A 4,904,408, US-A 4,846,994 und US-A 4,849,126) oder modifizierte Cellulosen wie z. B. Methylcellulose, Hydroxypropylcellulose oder Carboxymethylcellulose.

### Farbübertragungsinhibitoren

Als Farbübertragungsinhibitoren werden beispielsweise Homo- und Copolymere des Vinylpyrrolidons, des Vinylimidazols, des Vinyloxazolidons und des 4-Vimylpyridin-N-oxids mit Molmassen von 15.000 bis 100.000 sowie vernetzte feinteilige Polymere auf Basis dieser Monomeren eingesetzt. Die hier genannte Verwendung solcher Polymere ist bekannt, vgl. DE-B- 22 32 353, DE-A-28 14 287, DE-A-28 14 329 und DE-A-43 16 023.

Geeignete Polyvinylpyridinbetaine sind z. B. in Tai, Formulating Detergents and Personal Care Products, AOCS Press, 2000, Seite 113 beschrieben.

Neben der Anwendung in Wasch- und Reinigungsmitteln für die Textilwäsche im Haushalt sind die erfindungsgemäß verwendbaren Waschmittelzusammensetzungen auch im Bereich der gewerblichen Textilwäsche und der gewerblichen Reinigung einsetzbar. In der Regel wird in diesem Einsatzbereich Peressigsäure als Bleichmittel eingesetzt, die als wäßrige Lösung der Waschflotte zugesetzt wird.

### Verwendung in Teztilwaschmitteln

Ein typisches erfindungsgemäßes pulver- oder granulatförmiges Vollwaschmittel kann beispielsweise folgende Zusammensetzung aufweisen:
- 0,5 bis 50 Gew.-%, vorzugsweise 5 bis 30 Gew.-%, mindestens eines anionischen und/- oder nichtionischen Tensids, einschließlich der erfindungsgemäßen Gemische,
- 0,5 bis 60 Gew.-%, vorzugsweise 15 bis 40 Gew.-%, mindestens eines anorganischen Builders,
- 0 bis 20 Gew.-%, vorzugsweise 0,5 bis 8 Gew.-%, mindestens eines organischen Cobuilders,
- 2 bis 35 Gew.-%, vorzugsweise 5 bis 30 Gew.-%, eines anorganischen Bleichmittels,
- 0,1 bis 20 Gew.-%, vorzugsweise 0,5 bis 10 Gew.-%, eines Bleichaktivators, gegebenenfalls in Abmischung mit weiteren Bleichaktivatoren,
- 0 bis 1 Gew.-%, vorzugsweise bis höchstens 0,5 Gew.-%, eines Bleichkatalysators,
- 0 bis 5 Gew.-%, vorzugsweise 0 bis 2,5%, eines polymeren Farbübertragungsinhibitors,
- 0 bis 1,5 Gew.-%, vorzugsweise 0,1 bis 1,0 Gew.-%, Protease,
- 0 bis 1,5 Gew.-%, vorzugsweise 0,1 bis 1,0 Gew.%, Lipase,
- 0 bis 1,5 Gew.-%, vorzugsweise 0,2 bis 1,0% Gew.-% eines Soil-Release-Polymers,
ad 100% übliche Hilfs- und Begleitstoffe und Wasser.

Vorzugsweise in Waschmitteln eingesetzte anorganische Builder sind Natriumcarbonat, Natriumhydrogencarbonat, Zeolith A und P sowie amorphe und kristalline Na-Silikate sowie Schichtsilikate.

Vorzugsweise in Waschmitteln eingesetzte organische Cobuilder sind Acrylsäure/Maleinsäure-Copolymere, Acrylsäure/Maleinsäure/Vinylester- Terpolymere und Citronensäure.

Vorzugsweise in Waschmitteln eingesetzte anorganische Bleichmittel sind Natriumperborat und Natriumcarbonat-Perhydrat.

Vorzugsweise in Waschmitteln eingesetzte anionische Tenside sind lineare und leicht verzweigte Alkylbenzolsulfonate (LAS), Fettalkoholsulfate/ethersulfate und Seifen.

Vorzugsweise in Waschmitteln eingesetzte Enzyme sind Protease, Lipase, Amylase und Cellulase. Von den handelsüblichen Enzymen werden dem Waschmittel in der Regel Mengen von 0,05 bis 2,0 Gew.-%, vorzugsweise 0,2 bis 1,5 Gew.-%, des konfektionierten Enzyms zugesetzt. Geeignete Proteasen sind z.B Savinase, Desazym und Esperase. Eine geeignete Lipasen ist z. B. Lipolase. Eine geeignete Cellulase ist z. B. Celluzym.

Vorzugsweise in Waschmitteln eingesetzte Vergrauungsinhibitoren und Soil-Release-Polymere sind Pfropfpolymere von Vinylacetat auf Polyethylenoxid der Molmasse 2.500-8.000 im Gewichtsverhältnis 1,2:1 bis 3,0:1, Polyethylenterephthalate/Oxyethylenterephthalate der Molmasse 3.000 bis 25.000 aus Polyethylenoxiden der Molmasse 750 bis 5.000 mit Terephthalsäure und Ethylenoxid und einem Molverhältnis von Polyethylenterephthalat zu Polyoxyethylenterephthalat von 8:1 bis 1:1 sowie Blockpolykondensate gemäß DE-A-44 03 866.

Vorzugsweise in Waschmitteln eingesetzte Farbübertragungsinhibitoren sind lösliche NVP-Homopolymere und/oder Vinylpyrrolidon- und Vinylimidazol-Copolymere mit Molmassen über 5.000.

Die Waschmittel liegen häufig in fester, pulverförmiger Form vor, und enthalten dann in der Regel zusätzlich übliche Stellmittel, die ihnen eine gute Rieselfähigkeit, Dosierbarkeit und Löslichkeit verleihen und die das Zusammenbacken und Stauben verhüten, wie Natriumsulfat oder Magnesiumsulfat.

Die erfindungsgemäßen pulver- oder granulatförmigen Waschmittel können bis zu 60 Gew.-% anorganischer Stellmittel enthalten. Vorzugsweise sind die erfindungsgemäßen Waschmittel aber arm an Stellmitteln und enthalten nur bis zu 20 Gew.-%, besonders bevorzugt nur bis 8 Gew.-% an Stellmitteln.

Die erfindungsgemäßen Waschmittel können unterschiedliche Schüttdichten im Bereich von 300 bis 1.200, insbesondere 500 bis 950g/l, besitzen. Moderne Kompaktwaschmittel besitzen in der Regel hohe Schüttdichten und zeigen einen Granulataufbau. Kompakt- oder Ultra-Kompaktwaschmittel sowie Extrudate weisen ein Schüttgewicht > 600 g/l auf. Diese gewinnen immer mehr an Bedeutung.

Sofern sie in flüssiger Form eingesetzt werden sollen, können sie als wäßrige Mikroemulsionen, Emulsionen oder Lösungen vorliegen. In flüssigen Waschmitteln können zusätzlich Lösungsmittel wie Ethanol, i-Propanol, 1,2-Propylenglykol, oder Butylglykol verwendet werden.

Bei gelförmigen erfindungsgemäßen Waschmitteln können zusätzlich Verdicker, wie z. B. Polysaccharide und/oder schwach vernetzte Polycarboxylate (beispielsweise Carbopol® der Fa. Goodrich) eingesetzt werden.

Bei tablettenförmigen Waschmitteln werden zusätzlich Tablettierhilfsmittel wie z. B. Polyethylenglykole mit Molmassen > 1000 g/mol, Polymerdispersionen, und Tablettensprengmittel wie Cellulosederivate, vernetztes Polyvinylpyrrolidon, vernetzte Polyacrylate oder Kombinationen aus Säuren, z. B. Citronensäure + Natriumbicarbonat, um nur einige zu nennen, benötigt.

Ein weiterer Gegenstand der vorliegenden Erfindung ist die Verwendung der Gemische bei der Herstellung von Waschmitteln.

Noch ein weiterer Gegenstand der Erfindung ist ein Waschverfahren unter Einsatz eines erfindungsgemäßen Waschmittels.

Die Erfindung wird anhand der nachstehenden Beispiele näher erläutert.

### Herstellungsbeispiele

Der Alkohol und KOH (fein gepulvert) wurden vermischt und bei 80°C und 40 mbar über 1 Stunde entwässert. Das Reaktionsprodukt wurde in einen Autoklaven gegeben, der Autoklav 2 mal mit Stickstoff inertisiert und dann auf 120°C erwärmt. Innerhalb von 15 Minuten wurde Ethylenoxid bis zu einem Maximaldruck von 1 bar zudosiert. Man hielt 5 min bei diesem Druck, steigerte den Druck dann durch Zugabe von Ethylenoxid innerhalb 60 min auf 3 bar, hält 5 Stunden bei diesem Druck und steigerte schließlich den Druck bis auf 6 bar. Bei der letzten Dosierung wurde nur soviel Ethylenoxid zugegeben, bis die unten angegebene Menge Ethylenoxid erreicht war. Der Druck wurde dann durch Zudosierung von Stickstoff bei 6 bar gehalten. Nach weiteren 10 Stunden Reaktionszeit wurde auf Zimmertemperatur abkühlen gelassen, und der Reaktionsaustrag wurde ausgefüllt. Am Rotationsverdampfer wurden flüchtige Anteile bei 30 mbar und 80°C entfernt.

### Beispiel 1

### 2-Propylheptanol + 5 EO

Es wurden 474 g 2-Propylheptanol (3,0 mol), 661 g Ethylenoxid (15,0 mol) und 2,3 g KOH verwendet.

### Beispiel 2

### 2-Propylheptanol + 7 EO

Es wurden 474 g 2-Propylheptanol (3,0 mol), 925 g Ethylenoxid (21,0 mol) und 2,8 g KOH verwendet.

### Beispiel 3

### 2-Propylheptanol + 10 EO

Es wurden 474 g 2-Propylheptanol (3,0 mol), 1.322 g Ethylenoxid (30,0 mol) und 3,6 g KOH verwendet.

### Beispiel 4

### iso-C13-Alkohol 5 EO

Es wurden 401 g iso-C13-Alkohol (2,0 mol), 441 g Ethylenoxid (10,0 mol) und 1,7 g KOH verwendet.

### Beispiel 5

### iso-C13-Alkohol 7 EO

Es wurden 401 g iso-C13-Alkohol (2,0 mol), 617 g Ethylenoxid (14,0 mol) und 2,0 g KOH verwendet.

### Beispiel 6

### iso-C13-Alkohol 11 EO

Es wurden 401 g iso-C13-Alkohol (2,0 mol), 969 g Ethylenoxid (22,0 mol) und 2,7 g KOH verwendet.

### Anwendungsbeispiele

| Waschbedingungen | Primärwäsche | |
|---|---|---|
| Gerät | Launder-o-meter von Atlas, Chicago USA | |
| Waschflotte | 250 ml | |
| Waschdauer | 30 min. bei angegebener Temperatur (einschließlich Aufheizzeit) | |
| Waschmitteldosierung | 4,5 g/l | |
| Wasserhärte | 3 mmol/l | Ca: Mg 4 : 1 |
| Flottenverhältnis | 1:12,5 | |

| Prüfgewebe | | Hersteller | |
|---|---|---|---|
| wfk 10C | Wollfett/Pigment auf Baumwolle | wfk | Testgewebe GmbH, Brüggen, Deutschland |
| wfk 10D | Hautfett/Pigment auf Baumwolle | wfk | Testgewebe GmbH, Brüggen, Deutschland |
| wfk 20D | Hautfett/Pigment auf Mischgewebe | wfk | Testgewebe GmbH, Brüggen, Deutschland |
| wfk 10PF | Pflanzenfett/Pigment auf Baumwolle | wfk | Testgewebe GmbH, Brüggen, Deutschland |
| EMPA 101 | Olivenöl/Ruß auf Baumwolle | EMPA | Testmaterialien, St. Gallen, Schweiz |

Die gewaschenen Prüfgewebe werden mit einem Photometer von Datacolor (Ekepho 2000) vermessen. Angegeben ist die Schmutzentfernung in Prozent. Das Primärwaschvermögen ist umso besser, je höher die Schmutzentfernung ist. Waschformulierung

| | |
|---|---|
| Natriumcarbonat | 12 % |
| Natritunperborat-monohydrat | 14,4 % |
| Natriumsilikat | 3 % |
| Natriumsulfat | 4 % |
| Seife | 0,5 % |
| Copolymer AS/MS 7 : 3 | 5 % |
| TAED | 4 % |
| Carboxymethylcellulose | 1,2 % |
| Zeolith A | 30 % |
| erfindungsgemäße Tenside | wie angegeben |
| Wasser | Rest auf 100% |

| | |
|---|---|
| R = Remissionswert bei 460 nm | |

Die Ergebnisse der Waschversuche sind in der nachstehenden Tabelle zusammengefasst.

**Tabelle**

| | | | | | Gewebetyp wfk 10C | wfk 10D | wtk 20 D | wfk 10 PF | EMPA 101 |
|---|---|---|---|---|---|---|---|---|---|
| Tensid 1 aus Beispiel | Tensid 2 aus Beispiel | Menge Tensid 1 [%] | Menge Tensid 2 [%] | Wasch temperatur | Schmutz- entf. in % | Schmutz- entf. in % | Schmutz- entf. in % | Schmutz- entf. in % | Schmutz- entf. in % |
| 1 | - | 6 | - | 25°C | 5,2 | 0,4 | 4,0 | - | - |
| 1 | 4 | 3 | 3 | 25°C | - | 19,9 | 26,2 | - | - |
| 1 | 5 | 3 | 3 | 25°C | 7,7 | 20,4 | 22,5 | - | - |
| 1 | - | 6 | - | 40°C | 6,1 | 0,4 | 3,5 | 6,9 | 3,3 |
| 1 | 4 | 3 | 3 | 40°C | 23,2 | 29,5 | 32,5 | 26,3 | 7,7 |
| 1 | 5 | 3 | 3 | 40°C | 17,6 | 24,6 | 30,8 | - | 11,1 |
| 1 | 6 | 4 | 2 | 40°C | 25,0 | 30,6 | 31,1 | 28,7 | 6,3 |
| 1 | - | 6 | - | 60°C | 13,1 | 0,7 | 5,9 | 15,8 | 7,5 |
| 1 | 4 | 3 | 3 | 60°C | 40,8 | 48,9 | 27,7 | 29,4 | - |
| 1 | 5 | 3 | 3 | 60°C | 28,1 | 32,2 | 59,5 | - | 11,3 |
| 1 | 6 | 4 | 2 | 60°C | 23,4 | 40,8 | 31,7 | 40,1 | 16,9 |
| 2 | - | 6 | - | 40°C | 4,9 | 26,0 | 10,3 | 5,7 | 9,2 |
| 2 | 4 | 3 | 3 | 40°C | 15,8 | 46,1 | 21,5 | 11,8 | 12,2 |
| 2 | 5 | 3 | 3 | 40°C | 19,4 | 37,9 | 36,8. | 18,6 | 15,1 |
| 2 | - | 6 | - | 60°C | 17,9 | 32,0 | 7,7 | 6,9 | 10,7 |
| 2 | 4 | 3 | 3 | 60°C | 29,9 | 40,8 | 18,5 | 18,8 | 11,6 |
| 2 | 5 | 3 | 3 | 60°C | 25,6 | 46,6 | 40,0 | 26,4 | 17,4 |
| 3 | - | 6 | - | 40°C | 7,6 | 2,1 | 1,9 | 7,5 | 7,0 |
| 3 | 4 | 3 | 3 | 40°C | 15,5 | 19,2 | 16,4 | - | 10,8 |
| 3 | 5 | 3 | 3 | 40°C | 19,4 | 37,8 | 41,7 | 31,6 | 8,1 |
| 3 | - | 6 | - | 60°C | 14,7 | 0,3 | 7,2 | 13,5 | 10,8 |
| 3 | 4 | 3 | 3 | 60°C | 28,5 | 18,8 | 35,6 | - | 21,2 |
| 3 | 5 | 3 | 3 | 60°C | 25,3 | 40,1 | 34,5 | 30,0 | 17,2 |

Wie aus den in der Tabelle zusammengefassten Ergebnissen hervorgeht, führt der Einsatz des erfindungsgemäßen Alkoxylatgemisches zu einer deutlichen Verbesserung der Primärwaschwirkung im Waschmittel.

### Beispiel 7

### Herstellbeispiel: DMC-Katalysator

In einem Rührkessel mit einem Volumen von 301, ausgestattet mit einem Propellerrührer, Tauchrohr für die Dosierung, pH-Sonde und Streulicht-Sonde, wurden 16000 g wässrige Hexacyanocobaltsäure (Cobalt-Gehalt: 9 g/l) vorgelegt und unter Rühren auf 50°C erwärmt. Anschließend wurden unter Rühren mit einer Rührleistung von 0,4 W/1 9224 g wässrige Zinkacetat-Dihydrat-Lösung (Zink-Gehalt: 2,6 Gew.-%), welche auf ebenfalls 50°C temperiert war, innerhalb von 15 Minuten zugefahren.

Zu dieser Fällsuspension wurden 351 g Pluronic® PE 6200 (BASF AG) zugesetzt und die Mischung weitere 10 Minuten gerührt.

Anschließend wurden weitere 3690 g wässrige Zinkacetat-Dihydrat-Lösung (Zink-Gehalt: 2,6 Gew.-%) unter Rühren mit einer Rührenergie von 1 W/1 innerhalb 5 Minuten zudosiert.

Die Suspension wurde zwei Stunden nachgerührt. Der pH-Wert fiel in dieser Zeit von 4,02 auf 3,27 und blieb dann konstant. Die so erhaltene Fällsuspension wurde anschließend abfiltriert und auf dem Filter mit dem 6-fachen Kuchenvolumen an Wasser gewaschen.

Der feuchte Filterkuchen wurde getrocknet und mittels Spalt-Rotor-Mühle in Tridecanol® N dispergiert. Die dabei erhaltene Suspension hatte einen Multimetallcyanidgehalt von 5 Gew.-%.

### 2-Propylheptanol + 5 EO, 25 ppm DMC

474 g (3,0 Mol) 2-Propyl-Heptanol-1 (Isomerengemisch aus 87% 2-Propylheptanol-1, 11 % 2-Propyl-4-methylhexanol-1, < 1% 2-Propyl-S-methylhexanol-1) und 0,567 g einer 5%-igen Suspension von Doppelmetallcyanid in 2-Propylheptanol-Isomerengemisch (25 ppm bezogen auf das Produkt) als Katalysator wurden bei einer Temperatur von 80 °C und ca. 1 mbar entwässert, anschließend in einem 21 Druckautoklaven vorgelegt, dreimal mit Stickstoff gespült und danach auf 120 °C erhitzt. Nach Erreichen der Temperatur wurden in 1,05 Stunden 660 g (15 Mol) Ethylenoxid kontinuierlich bei einem Druck von 0,1 bis 3,7 bar zudosiert (Druckrampe 6 bar/ 90 min). Nach vollständiger Oxidzugabe wurde bis zur Druckkonstanz nachreagieren lassen (20 Minuten), danach auf 80 °C abgekühlt, dreimal mit Stickstoff gespült und entleert. Das so erhaltene Produkt wurde bei 80 °C am Rotationsverdampfer unter Vakuum (< 30 mbar) entgast (Reaktionsprodukt nicht filtriert).

### Beispiel 8

### 2-Propylheptanol + 8 EO, 25 ppm DMC

Die Umsetzung wurde analog Beispiel 7 durchgeführt mit 474 g (3,0 Mol) 2-Propylheptanol-Isomerengemisch, 0,77 g Doppelmetallcyanid-Suspension und 1060 g (24,0 Mol) Ethylenoxid.

Die gemäß Beispiel 7 und 8 erhaltenen Produkte wurden analog zu den gemäß Beispiel 1 bis 6 erhaltenen Produkten getestet.

## Patentansprüche

1. Alkoxylatgemisch, enthaltend
10 bis 90 Gew.-% mindestens eines Alkoxylats der allgemeinen Formel (I)
C₅H₁₁CH(C₃H₇)CH₂O(A)_{z}H (I)
mit der Bedeutung
A Ethylenoxy
x Zahl von 3 bis 12 und
10 bis 90 Gew.-% mindestens eines Alkoxylats der allgemeinen Formel (II)
CₘH₂ₘ₊₁O(A)ᵥ(B)_{w}H (II)
mit der Bedeutung
A Ethylenoxy
B C₃-C₁₀-Alkylenoxy oder Gemische davon,
wobei Gruppen A und B statistisch verteilt, alternierend oder in Form zweier oder mehrerer Blöcke in beliebiger Reihenfolge vorliegen können,
m ganze Zahl im Bereich von 12 bis 24,
v Zahl im Bereich von 3 bis 15,
w o, wobei im Alkoxylat der allge- Formel (I)
| | |
|---|---|
| 85 bis 96 Gew.-% | Alkoxylate A1, in denen C₅H₁₁ die Bedeutung n-C₅H₁₁ hat, und |
| 4 bis 15 Gew.-% | Alkoxylate A2, in denen C₅H₁₁ die Bedeutung C₂H₅CH(CH₃)CH₂ und/oder CH₃CH(CH₃)CH₂CH₂ hat, |
im Gemisch vorliegen.

2. Gemisch nach Anspruch 1 , **dadurch gekennzeichnet, dass** im Alkoxylat der allgemeinen Formel (II) m eine ganze Zahl im Bereich von 12 bis 18 ist.

3. Verfahren zur Herstellung von Alkoxylatgemischen nach Anspruch 1 oder 2, **dadurch gekennzeichnet, dass** Alkanole der allgemeinen Formen CₘH₂ₘ₋OH und 2-Propylheptanol mit der angegebenen Bedeutung für m mit Ethylenoxid unter Alkoxylierungsbedingungen umgesetzt werden und vor oder nach dem Alkoxylieren oder nach einem teliweisen Alkoxylieren miteinander gemischt werden, wobei die Alkoxylierung in Gegenwart einer Doppelmetallcyanid-Verbindung als Katalysator erfolgen kann.

4. Wasch- oder Reinigungsmittel, enthaltend ein Alkoxylatgemisch gemäß Anspruch 1 oder 2.

5. Wasch- oder Reinigungsmittel nach Anspruch 4, **dadurch gekennzeichnet, dass** das Alkoxylatgemisch in einer Menge von 0,01 bis 80 Gew.-%, bezogen auf das Wasch- oder Reinigungsmittel, vorliegt.

6. Verwendung eines Wasch- oder Reinigungsmittels gemäß Anspruch 4 oder 5 zum Waschen oder Reinigen von Textilien.

## Claims

1. An alkoxylate mixture comprising
10 to 90% by weight of at least one alkoxylate of the general formula (I)
C₅H₁₁CH(C₃H₇)CH₂O(A)ₓH (I)
where
A is ethyleneoxy,
x is a number from 3 to 12,
and
10 to 90% by weight of at least one alkoxylate of the general formula (II)
CₘH₂ₘ₊₁O(A)ᵥ(B)_{w}H where (II)
A is ethyleneoxy,
B is C₃-C₁₀-alkyleneoxy or mixtures thereof,
where groups A and B may be present randomly distributed, alternately or in the form of two or more blocks in any order,
m is an integer in the range from 12 to 24,
v is a number in the range from 3 to 15,
w is 0, where, in the alkoxylate of the general formula (I),
| | |
|---|---|
| 85 to 96% by weight | of alkoxylates A1, in which C₅H₁₁ has the meaning n-C₅H₁₁, and |
| 4 to 15% by weight | of alkoxylates A2, in which C5H11 has the meaning C2H5CH(CH3)CH2 and/or CH3CH(CH3)CH2CH2, |
are present in the mixture.

2. The mixture according to claim 1, wherein, in the alkoxylate of the general formula (II), m is an integer in the range from 12 to 18.

3. A process for the preparation of alkoxylate mixtures according to claim 1 or 2, which comprises reacting alkanols of the general formula CₘH₂ₘ₊₁OH and 2-propylheptanol with the given meaning for m with ethylene oxide under alkoxylation conditions, and mixing them together before or after alkoxylation or after partial alkoxylation, where the alkoxylation can be carried out in the presence of a double-metal cyanide compound as catalyst.

4. A detergent or cleaner comprising an alkoxylate mixture according to claim 1 or 2.

5. The detergent or cleaner according to claim 4, wherein the alkoxylate mixture is present in an amount of from 0.01 to 80% by weight, based on the detergent or cleaner.

6. The use of a detergent or cleaner according to claim 4 or 5 for the washing or cleaning of textiles.

## Revendications

1. Mélange d'alcoxylates, contenant
10 à 90 % en poids d'au moins un alcoxylate de formule générale (1)
C₅H₁₁CH(C₃H₇)CH₂O(A)ₓH (I)
avec la signification
A éthylèneoxy
x nombre de 3 à 12, et
10 à 90 % en poids d'au moins un alcoxylate de formule générale (II)
CₘH₂ₘ₊₁O(A)ᵥ(B)_{w}H (II)
avec la signification
A éthylèneoxy
B alkylèneoxy en C₃-C₁₀ ou leurs mélanges
les groupes A et B pouvant être présents répartis statistiquement, en alternance ou sous la forme de deux séquences ou plus dans un ordre quelconque,
m nombre entier dans la plage allant de 12 à 24,
v nombre dans la plage allant de 3 à 15,
w 0,
85 à 96 % en poids d'alcoxylates A1, dans lesquels C₅H₁₁ a la signification n-C₅H₁₁, et
4 à 15 % en poids d'alcoxylates A2, dans lesquels C₅H₁₁ a la signification C₂H₅CH (CH₃) CH₂ et/ou CH₃CH(CH₃)CH₂CH₂,
étant présents en mélange dans l'alcoxylate de formule générale (I).

2. Mélange selon la revendication 1, **caractérisé en ce que** m est un nombre entier dans la plage allant de 12 à 18 dans l'alcoxylate de formule générale (II).

3. Procédé de fabrication de mélanges d'alcoxylates selon la revendication 1 ou 2, **caractérisé en ce que** des alcanols de formule générale CₘH₂ₘ₊₁OH et du 2-propylheptanol avec la signification donnée pour m sont mis en réaction avec de l'oxyde d'éthylène dans des conditions d'alcoxylation et mélangés les uns avec les autres avant ou après l'alcoxylation ou après une alcoxylation partielle, l'alcoxylation pouvant avoir lieu en présence d'un composé de cyanure métallique double en tant que catalyseur.

4. Agent de lavage ou de nettoyage, contenant un mélange d'alcoxylates selon la revendication 1 ou 2.

5. Agent de lavage ou de nettoyage selon la revendication 4, **caractérisé en ce que** le mélange d'alcoxylates est présent en une quantité de 0,01 à 80 % en poids, par rapport à l'agent de lavage ou de nettoyage.

6. Utilisation d'un agent de lavage ou de nettoyage selon la revendication 4 ou 5 pour le lavage ou le nettoyage de textiles.
